# EUROPÄISCHE PATENTSCHRIFT

(11) **EP 1 741 423 B1**
(45) Veröffentlichungstag und Bekanntmachung des Hinweises auf die Patenterteilung: **13.08.2008**
(21) Anmeldenummer: 06011564.9
(22) Anmeldetag: 03.06.2006
(51) Int. Cl.: A61K 9/00, A61L 31/02

(54) **Arzneimitteldepot zur parenteralen, insbesondere intravaskulären Arzneimittelfreisetzung**
Drug depot for parenteral drug release, in particular intravascular drug release
Dépôt médicamenteux pour la libération parentérale, en particulier, pour la libération intravasculaire de médicaments

(30) Priorität: 04.07.2005 DE 102005031868
(43) Veröffentlichungstag der Anmeldung: 10.01.2007
(73) Patentinhaber: BIOTRONIK VI Patent AG, 6341 Baar (CH)
(72) Erfinder: Tittelbach, Michael, 90429 Nürnberg (DE); Bertsch, Torben, 90419 Nürnberg (DE); Bock, Ralf, Dr., 91056 Erlangen (DE)
(74) Vertreter: Lindner-Vogt, Karin L.

(56) Entgegenhaltungen:
- WO-A-20/05075005
- WO-A-20/06024488
- DE-A1- 19 856 983
- US-A1- 2001 044 651

## Beschreibung

### Technisches Gebiet

Die Erfindung betrifft ein Arzneimitteldepot zur parenteralen, insbesondere intravaskulären Arzneimittelfreisetzung.

### Hintergrund der Erfindung und Stand der Technik

Ein wichtiges Anliegen der pharmazeutischen Technologie ist die Entwicklung von Arzneiformen zur kontrollierten Arzneimittelfreisetzung, insbesondere zur Verzögerung der Freisetzung, um eine über einen größeren Zeitraum anhaltende und dabei gleichmäßige Dosierung eines Arzneimittels zu erzielen. Erreicht wird dies beispielsweise durch Bereitstellung von zunächst unwirksamen, erst im Körper aktivierbaren Vorstufen des Arzneimittels oder durch Zusatz von die Resorption des Arzneimittels verzögernden Hilfsstoffen. Weiterhin ist auch eine parenterale Applikation des Arzneimittels denkbar, zum Beispiel als injizierte Mikrokristallsuspension oder durch Implantation eines biokorrodierbaren Arzneimitteldepots, welches das einzubringende Arzneimittel allmählich freisetzt. Arzneimitteldepots sind millimeter- bis zentimetergroße Formlinge aus biokorrodierbaren Werkstoffen, etwa biokorrodierbaren Polymeren natürlichen Ursprungs, wie zum Beispiel Poly-DL-lactid-co-glycoliden, oder auf synthetischem Wege erhaltender Polymere. Das Arzneimittel wird mit dem biokorrodierbaren Werkstoff vermengt, als Beschichtung aufgebracht oder in eine Hülle aus dem Werkstoff eingebracht. Nach der Implantation erfolgt mit der Zersetzung des Werkstoffs einhergehend eine allmähliche Freisetzung des Arzneimittels.

So werden beispielsweise in der US-Patentanmeldung 2001/0044651 ausdehnbare, degradierbare Stents beschrieben, welche aus biokorrodierbaren Werkstoffen aufgebaut sein können, wie z.B. synthetische Polymere, Calcium Phosphat und Magnesiumlegierungen. Diese Stents können zusätzlich ein Arzneimittel enthalten der bei der Zersetzung des Werkstoffs freigesetzt wird.

Ein Freisetzungsverhalten des Arzneimittels hängt stark von der Wechselwirkung zwischen dem Arzneimittel und dem eingesetzten biokorrodierbaren Werkstoff sowie gegebenenfalls dessen Abbauprodukten ab. Mit anderen Worten, diese Art der Wechselwirkung wird zur Optimierung der parenteralen, insbesondere intravaskulären Arzneimittelfreisetzung zu berücksichtigen sein. Auch muss der Ort der Freisetzung im Körper beachtet werden, da sich lokal deutliche Unterschiede in den die Freisetzung beeinflussenden Faktoren, wie Hydrophilie/Hydrophobie, pH-Wert, Strömungsverhältnisse oder Sauerstoffgehalt zeigen können.

### Zusammenfassung der Erfindung

Aufgabe der vorliegenden Erfindung ist es, ein für die parenterale, insbesondere intravaskuläre Freisetzung wenigstens eines Arzneimittels - das als Base oder als dazu korrespondierendes protoniertes Salz vorliegen kann, wobei die Base einen pKb-Wert im Bereich von 2 bis 6 hat - geeignetes Arzneimitteldepot bereitzustellen.

Das erfindungsgemäße Arzneimitteldepot zur parenteralen, insbesondere intravaskulären Arzneimittelfreisetzung, löst diese Aufgabe. Das Arzneimitteldepot enthält elementares Magnesium in einer biokorrodierbaren Form. Der Erfindung liegt die Erkenntnis zugrunde, dass Arzneimittel, die als Base oder als dazu korrespondierendes protoniertes Salz vorliegen können, wobei die Base einen pKb-Wert im Bereich von 2 bis 6 hat, in physiologischer Umgebung zumindest in überwiegenden Teilen in der Salzform vorliegen. Liegt das Arzneimittel als Base vor, so steigt mit der Überführung des Arzneimittels in seine Salzform dessen Hydrophilie stark an, so dass die Freisetzung des Arzneimittels in physiologischer Umgebung beschleunigt wird. Liegt das Arzneimittel schon im Arzneimitteldepot in seiner Salzform vor, z. B. weil sich die Salze in der Regel besser verarbeiten lassen, weil die Basenform bei Raumtemperatur flüssige oder ölige Konsistenz hat, so wird ebenfalls das Arzneimittel sehr rasch in physiologischer Umgebung freigesetzt.

In physiologischer Umgebung, insbesondere in Blut, wird nun das erfindungsgemäß vorhandene elementare Magnesium zum stark basischen Magnesiumhydroxid abgebaut. Hierdurch wird erreicht, dass die Salzform des Arzneimittels wieder deprotoniert und das Gleichgewicht in Richtung der hydrophoberen Basenform des Arzneimittels verschoben wird. Mit dem Wechsel von der hydrophilen Salzform des Arzneimittels hin zur hydrophoben Basenform des Arzneimittels einhergehend lässt sich das Resorptionsverhalten im Körper beeinflussen. Ist das Arzneimitteldepot zur intravaskulären Arzneimittelfreisetzung angepasst, so kann auf diese Weise eine rasche Freisetzung der hydrophilen Salzform des Arzneimittels ins Blut verhindert oder zumindest gemindert werden. Liegt das Arzneimitteldepot zudem an der Gefäßwand an, so wird angenommen, dass eine zielgerichtete Applikation in Richtung der Gefäßwand möglich ist, denn diese hat gegenüber dem im Lumen des Gefäßes fließenden Blut einen deutlich hydrophoberen Charakter, so dass ein Eindringen der hydrophoberen Basenform des Arzneimittels unterstützt werden sollte.

Der pKb-Wert ist definiert als der negative dekadische Logarithmus des Dissoziationsgleichgewichts B+ H₂O ⇄ BH⁺ + OH⁻ der elektrolytischen Dissoziation, wobei B für Base steht. Er wird dazu benutzt, die Basizität des Arzneimittels bei Raumtemperatur auszudrücken.

Unter Arzneimittel wird vorliegend ein Stoff verstanden, der als Therapeutikum zur Beeinflussung von Zuständen oder Funktionen des Körpers eingesetzt werden kann. Das Arzneimittel weist eine basische Funktionalität auf und ist vorzugsweise eine organische Stickstoffverbindung, könnte aber auch eine Schwefel- oder Phosphorverbindung sein. Das Arzneimittel zeichnet sich weiterhin dadurch aus, dass es in physiologischer Umgebung, insbesondere im Blut, durch Protonierung zumindest in überwiegenden Teilen in Form des korrespondierenden Salzes vorliegt. Der normale pH-Wert im arteriellen Blut liegt bei etwa 7,40 und in venösem Blut bei etwa 7,37. Für die erfindungsgemäßen Zwecke eignen sich Arzneimittel mit einem pKb-Wert im Bereich von 2 bis 6, vorzugsweise im Bereich von 3 bis 6.

Arzneimittel vorgenannten Typs umfassen vorzugsweise aliphatische Amine. Insbesondere ist bevorzugt, wenn das Arzneimittel Verapamil (5-[N-(3,4-Dimethoxyphenethyl)-N-methylamino]-2-(3,4-dimethoxyphenyl)-2-isopropylvaleronitril) ist. Verapamil ist ein Calcium-Antagonist und weist einen pKb-Wert von 5,4 auf. Zur Herstellung des Arzneimitteldepotes wird Verapamil vorzugsweise als Hydrochlorid eingesetzt, da sich das Salz gegenüber dem bei Raumtemperatur öligen Substrat des nicht-ionischen Amins besser verarbeiten lässt.

Eine biokorrodierbare Form des elementaren Magnesiums liegt dann vor, wenn nach der Implantation des Arzneimitteldepots durch körpereigene Prozesse ein Abbau des Magnesiums stattfindet. Der Abbau wird insbesondere durch hydrolytische Prozesse bedingt sein, bei denen die starke Base Magnesiumhydroxid entsteht. Vorzugsweise ist die Biokorrosion des elementaren Magnesiums nach spätestens 6 Monaten, besonders bevorzugt 3 Monaten, zu mehr als 90 Gew.%, bezogen auf den Anteil des insgesamt vor der Implantation vorhandenen elementaren Magnesiums, abgeschlossen.

Weiterhin ist bevorzugt, wenn das elementare Magnesium Bestandteil einer biokorrodierbaren Magnesiumlegierung ist. Unter Magnesiumlegierung wird vorliegend eine Legierung verstanden, in der Magnesium einen Gewichtsanteil von mindestens 50 % hat. Die biokorrodierbare Magnesiumlegierung ist vorzugsweise eine Legierung der Zusammensetzung Yttrium 3,7 - 5,5 Gew.%, seltene Erden 1,5 - 4,4 Gew.% und Rest < 1 Gew.%, wobei Magnesium den auf 100 Gew.% fehlenden Anteil an der Legierung einnimmt. Die Magnesiumlegierungen zeichnen sich durch ihre leichte Verarbeitbarkeit und ihr günstiges Degradationsverhalten für die erfindungsgemäßen Zwecke aus.

Durch Änderung eines molaren Verhältnisses des mit der Zersetzung von Magnesium entstehenden Magnesiumhydroxids zum Arzneimittel kann das Freisetzungsverhalten beeinflusst werden. Wird der Anteil des Magnesiumhydroxids im Vergleich zum Arzneimittel erhöht, so wird die Freisetzung in einem hydrophilen Medium verzögert. Es deutet sich an, dass für den vorliegenden Einsatzzweck ein molares Verhältnis des mit der Zersetzung von Magnesium entstehenden Magnesiumhydroxids zum Arzneimittel vorzugsweise im Bereich von 1 : 1 bis 50 : 1, besonders bevorzugt im Bereich von 1 : 1 bis 10 : 1 liegen sollte, und zwar (i) über den Zeitraum, in dem das Gleichgewicht zwischen der hydrophilen Salzform des Arzneimittels hin zur hydrophoben Basenform des Arzneimittels zu verschieben ist, und (ii) lokal begrenzt auf den Ort der Retardation des Arzneimittels. Hierdurch kann sichergestellt werden, dass die Retardation des Arzneimittels nach Implantation des Arzneimitteldepots im erfindungsgemäßen Sinne verzögert erfolgt. Vorzugsweise erstreckt sich der vorgenannte Zeitraum über 1 bis 90 Tage, insbesondere 3 bis 30 Tage, beginnend mit der Implantation des Arzneimitteldepots in ein Blutgefäß. Unter dem Ort der Retardation wird der Bereich des Arzneimitteldepots verstanden, an dem das Körpermedium (in der Regel Blut) in Kontakt mit dem Arzneimittel tritt. Der Ort der Retardation wird in der Regel einer Grenzfläche entsprechen, die sich zwischen dem Körpermedium und dem festen oder öligen Arzneistoff bzw. einer den Arzneistoff enthaltenden Formulierung ergibt. Eine Konzentration des Magnesiumhydroxids und damit ein molares Verhältnis desselben zum Arzneimittel am Ort der Retardation hängt im Wesentlichen ab von (i) einer Geschwindigkeit, mit der das elementare Magnesium in Magnesiumhydroxid umgesetzt wird, und (ii) einer lokalen Nähe zwischen einem Abbauort des Magnesiums und dem Ort der Retardation. Die Geschwindigkeit der Umsetzung hängt hier wiederum vornehmlich von der Form ab, in der das elementare Magnesium vorliegt. So ist die Umsetzung einer biokorrodierbare Magnesiumlegierung gegenüber reinem Magnesium verzögert. Zudem bestimmt eine Zusammensetzung geeigneter Magnesiumlegierungen eine Geschwindigkeit der Umsetzung des enthaltenen Magnesiums, d. h. durch Anpassung der Legierungszusammensetzung kann eine Optimierung hin zum gewünschten Freisetzungsverhalten des Arzneimittels erfolgen. Je weiter entfernt der Abbauort des Magnesiums und der Ort der Retardation voneinander sind, umso höher muss der Umsatz des Magnesiums hin zum Magnesiumhydroxid sein, um die gewünschten Konzentrationsverhältnisse am Ort der Retardation einzustellen.

Ein zweiter Aspekt der Erfindung betrifft ein Verfahren zur Herstellung eines Arzneimitteldepots für die parenterale, insbesondere intravaskuläre Arzneimittelfreisetzung, das den folgenden Schritt umfasst:
- Vermengen oder Beschichten von elementarem Magnesium in einer biokorrodierbaren Form mit wenigstens einem Arzneimittel, das als Base oder als dazu korrespondierendes protoniertes Salz vorliegen kann, wobei die Base einen pKb-Wert im Bereich von 2 bis 6 hat, sowie gegebenenfalls weiteren Hilfsstoffen.

Das elementare Magnesium - sei es in Reinform oder als biokorrodierbare Magnesiumlegierung - kann demnach als Pulver oder Korn mit dem Arzneimittel und gegebenenfalls weiteren Hilfsstoffen vermengt werden. Das Gemenge wird - unter Zuhilfenahme von an sich bekannten Formungstechniken - zur Herstellung eines Formlings genutzt, der eine für den jeweiligen Einsatzzweck geeignete Formgebung und Beschaffenheit aufweist. Alternativ kann das Gemenge als Beschichtung auf einen Träger, beispielsweise einem endovaskulären Arzneimitteldepot oder einem Schrittmacher oder den Elektroden eines elektrotherapeutischen Implantats, aufgebracht werden. Die Beschichtung gemäß dieser Ausführungsform agiert dann als ein Arzneimitteldepot im erfindungsgemäßen Sinne. Hilfsstoffe können alle gängigen Zusatzstoffe bekannter pharmazeutischer Formulierungen umfassen, die die Herstellung des Formlings bzw. der Beschichtung unterstützen.

Nach einer weiteren Ausführungsform kann das Arzneimitteldepot einen massiven Körper aus Magnesium oder einer biokorrodierbaren Magnesiumlegierung umfassen. Nach dieser Variante wird das Arzneimittel - gegebenenfalls mit weiteren Hilfsstoffen - als Beschichtung auf den Körper aufgebracht. Dies kann beispielsweise durch Besprühen oder Eintauchen des Körpers in oder mit einer Lösung des Arzneimittels in einem geeigneten Lösungsmittel erfolgen.

Ein dritter Aspekt der Erfindung richtet sich auf die Verwendung von elementarem Magnesium - sei es in Reinform oder als biokorrodierbare Magnesiumlegierung - zur Herstellung eines Arzneimitteldepots zur parenteralen, insbesondere intravaskulären Freisetzung wenigstens eines Arzneimittels, das als Base oder als dazu korrespondierendes protoniertes Salz vorliegen kann, wobei die Base einen pKb-Wert im Bereich von 2 bis 6 hat. Nach Kenntnisstand der Anmelderin ist elementares Magnesium, vorzugsweise in Form einer biodegradierbaren Magnesiumlegierung, bisher nicht für Arzneimitteldepots zur parenteralen Applikation basischer Arzneimittel genutzt wurden.

### Kurzbeschreibung der Figuren

- Fig. 1: zeigt stark schematisiert ein Arzneimitteldepot zum vaskulären Einsatz;
- Fig. 2: zeigt einen Schnitt durch einen Teil eines Arzneimitteldepots nach einer ersten Variante; und
- Fig. 3: zeigt einen Schnitt durch einen Teil eines Arzneimitteldepots nach einer zweiten Variante.

### Detaillierte Beschreibung anhand von Ausführungsbeispielen

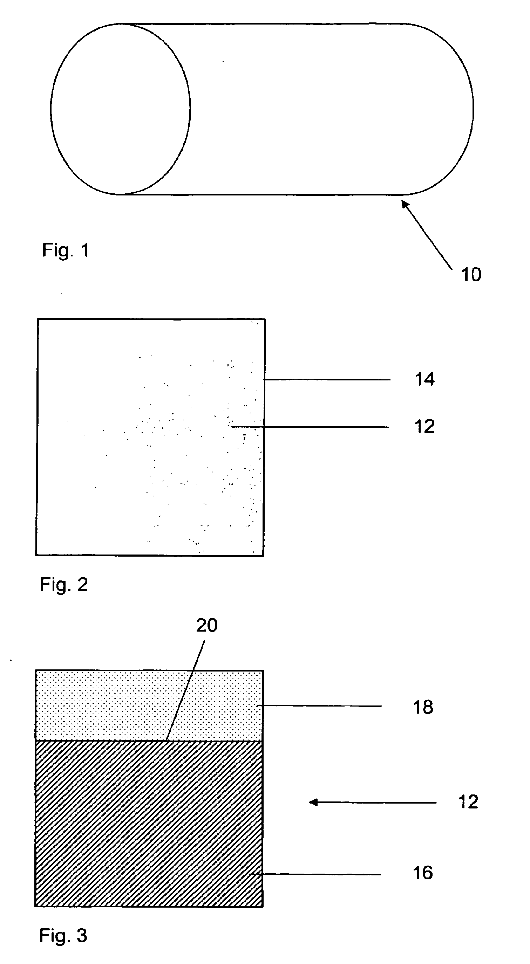

Fig. 1 zeigt ein Arzneimitteldepot 10 zur parenteralen, intravaskulären Arzneimittelfreisetzung, das aus einer Magnesiumlegierung der Zusammensetzung Yttrium 3,7 bis 5,5 Gew.%, seltene Erden 1,5 bis 4,4 Gew.% und Rest < 1 Gew.%, wobei Magnesium den auf 100 Gew.% fehlenden Anteil an der Legierung einnimmt, geformt ist. Das Arzneimitteldepot 10 ist zum Einsatz in einem Blutgefäß optimiert, d. h. es weist einen röhrenförmigen Grundkörper auf, durch dessen Innern Blut strömen kann. Das Arzneimitteldepot 10 lässt sich mit geeigneten Mitteln, z.B. kleinen Haken oder Stacheln, in einer Gefäßwand des Blutgefäßes verankern.

Das Arzneimitteldepot 10 wird mit einer etwa 1 molaren Lösung des Hydrochlorids von Verapamil in Aceton benetzt und das Lösungsmittel anschließend unter vermindertem Druck abgedampft. Der Prozess wir so oft wiederholt, bis ein molares Verhältnis des Magnesiums zum Hydrochlorid von Verapamil etwa im Bereich von 30 : 1 bis 50 : 1 liegt. Auch nach der Beschichtung ist eine Oberfläche des massiven Grundkörpers aus der Magnesiumlegierung noch zugänglich, so dass ein Abbau der Magnesiumlegierung unter Freisetzung von Magnesiumhydroxid stattfinden kann. Für das angegebene Verhältnis von Magnesium zum Arzneimittel wird angenommen, dass sich hierdurch ein molares Verhältnis des mit der Zersetzung von Magnesium entstehenden Magnesiumhydroxids zum Arzneimittel im gewünschten Sinne einstellen lässt. Ein Zeitraum, in dem das Gleichgewicht zwischen der hydrophilen Salzform von Verapamil hin zur hydrophoben Basenform von Verapamil zu verschieben ist, soll etwa 3 bis 30 Tage, beginnend mit der Implantation des Arzneimitteldepots in ein Blutgefäß, betragen. Das molare Verhältnis von Magnesiumhydroxid zum Verapamil sollte dabei am Ort der Retardation über den genannten Zeitraum bei 1 : 1 bis 10 : 1 liegen.

Die Fig. 2 und 3 zeigen jeweils einen Schnitt durch einen Teil 12 eines Arzneimitteldepots nach zwei Varianten. Eine Geometrie des dargestellten Teils 12 ist nur von untergeordneter Bedeutung und wird entsprechend den baulichen Notwendigkeiten des Arzneimitteldepots anzupassen sein. Vorliegend soll nur der grundsätzliche Aufbau demonstriert werden.

In der Variante nach Fig. 2 besteht der dargestellte Teil 12 des Arzneimitteldepots aus einem weitgehend homogenen Gemenge eines Arzneimittels (angedeutet durch die Körner) und elementaren Magnesiums als dieses Arzneimittel umgebende Matrix (angedeutet durch die Freiräume zwischen den Körnern). Das Arzneimittel ist beispielsweise Verapamil und zwar in Form seines Hydrochlorids. Eine Oberfläche 14 steht nach der Implantation in Kontakt mit dem Körpermedium, also in der Regel Blut. Hierdurch wird an der Oberfläche 14 eine Umsetzung des Magnesiums hin zum Magnesiumhydroxid erfolgen, so dass eine lokale Konzentration an Magnesiumhydroxid an einer Grenzfläche zwischen der Oberfläche 14 des Arzneimitteldepots und dem Köpermedium erhöht ist. Dies führt wiederum dazu, dass das Verapamil vom salzförmigen Hydrochlorid hin zu seiner öligen nicht-ionischen Form überführt wird. Letztere ist in einem wässrigen Medium wie Blut nur noch in einem vernachlässigbarem Umfang löslich.

In der Variante nach Fig. 3 ist der dargestellte Teil 12 des Arzneimitteldepots zweigliedrig ausgeführt. Ein massiver Grundkörper 16 aus einer biokorrodierbaren Magnesiumlegierung wird mit einer porösen Schicht 18, die das Arzneimittel enthält, überdeckt. Das Arzneimittel kann beispielsweise wieder Verapamil sein, das als Hydrochlorid vorliegt. Der Schicht 18 sind weiterhin übliche Trägerstoffe für Arzneimittel zugesetzt, wobei eine hinreichende Porosität der Schicht 18 zu gewährleisten ist. Nach der Implantation kann das Körpermedium nur über die Schicht 18 zum Grundkörper 16 vordringen, d. h. die in Fig. 3 dargestellten Seitenoberflächen und die Unterseite des massiven Grundkörpers 16 sind baulich entsprechend unzugänglich (z. B. ist das Arzneimitteldepot kugelförmig mit einem Innen liegenden Kern als Grundkörper ausgeführt). Eine Oberfläche 20 des Grundkörpers 16 steht nach der Implantation in Kontakt mit dem Körpermedium. Hierdurch wird an der Oberfläche 20 eine Umsetzung des Magnesiums hin zum Magnesiumhydroxid erfolgen. Dieses wird sich durch Diffusion in der Schicht 18 verteilen, so dass eine lokale Konzentration an Magnesiumhydroxid an einer Grenzfläche zwischen einer Oberfläche der Schicht 18, die das Hydrochlorid von Verapamil enthält, und dem Köpermedium erhöht ist. Dies führt wiederum dazu, dass das Verapamil vom salzförmigen Hydrochlorid hin zur öligen nicht-ionischen Form überführt wird. Letztere ist in einem wässrigen Medium wie Blut nur noch in einem vernachlässigbarem Umfang löslich.

## Patentansprüche

1. Arzneimitteldepot zur parenteralen, insbesondere intravaskulären Arzneimittelfreisetzung, enthaltend (i) wenigstens ein Arzneimittel, das als Base oder als dazu korrespondierendes protoniertes Salz vorliegen kann, wobei die Base einen pKb-Wert im Bereich von 2 bis 6 hat, und (ii) elementares Magnesium in einer biokorrodierbaren Form.

2. Arzneimitteldepot nach Anspruch 1, **dadurch gekennzeichnet, dass** das elementare Magnesium Bestandteil einer Magnesiumlegierung ist.

3. Arzneimitteldepot nach den Ansprüchen 1 oder 2, **dadurch gekennzeichnet, dass** das Arzneimittel Verapamil ist.

4. Verwendung von elementarem Magnesium in einer biokorrodierbaren Form zur Herstellung eines Arzneimitteldepots zur parenteralen, insbesondere intravaskulären Freisetzung wenigstens eines Arzneimittels, das als Base oder als dazu korrespondierendes protoniertes Salz vorliegen kann, wobei die Base einen pKb-Wert im Bereich von 2 bis 6 hat.

5. Verfahren zur Herstellung eines Arzneimitteldepots für die parenterale, insbesondere intravaskuläre Arzneimittelfreisetzung, umfassend den Schritt:
- Vermengen oder Beschichten von elementarem Magnesium in einer biokorrodierbaren Form mit wenigstens einem Arzneimittel, das als Base oder als dazu korrespondierendes protoniertes Salz vorliegen kann, wobei die Base einen pKb-Wert im Bereich von 2 bis 6 hat, sowie gegebenenfalls weiteren Hilfsstoffen.

## Claims

1. A pharmaceutical depot for parenteral, in particular intravascular pharmaceutical release, containing (i) at least one pharmaceutical, which may be provided as a base or as the protonated salt corresponding thereto, the base having a pKb value in the range from 2 to 6, and (ii) elementary magnesium in a biocorrodible form.

2. The pharmaceutical depot according to Claim 1, **characterized in that** the elementary magnesium is a component of a magnesium alloy.

3. The pharmaceutical depot according to Claims 1 or 2, **characterized in that** the pharmaceutical is verapamil.

4. A use of elementary magnesium in a biocorrodible form for producing a pharmaceutical depot for parenteral, in particular intravascular release of at least one pharmaceutical, which may be provided as a base or as the protonated salt corresponding thereto, the base having a pKb value in the range from 2 to 6.

5. A method for producing a pharmaceutical depot for parenteral, in particular intravascular pharmaceutical release, comprising the following step:
- admixing or coating elementary magnesium in a biocorrodible form with at least one pharmaceutical, which may be provided as the base or as the protonated salt corresponding thereto, the base having a pKb value in the range from 2 to 6, as well as possibly further adjuvants.

## Revendications

1. Dépôt de médicament pour la libération de médicament parentérale, en particulier intravasculaire, contenant (i) au moins un médicament, qui peut être présent sous forme de base ou sous forme de sel protoné correspondant à cette base, la base ayant une valeur pKb de l'ordre de 2 à 6, et (ii) du magnésium élémentaire dans une forme biocorrodable.

2. Dépôt de médicament selon la revendication 1, **caractérisé en ce que** le magnésium élémentaire est un composant d'un alliage de magnésium.

3. Dépôt de médicament selon les revendications 1 ou 2, **caractérisé en ce que** le médicament est du Vérapamil.

4. Utilisation de magnésium élémentaire dans une forme biocorrodable pour la fabrication d'un dépôt de médicament pour la libération parentérale, en particulier intravasculaire, d'au moins un médicament, qui peut être présent sous forme de base ou de sel protoné correspondant à cette base, la base ayant une valeur pKb de l'ordre de 2 à 6.

5. Procédé pour la fabrication d'un dépôt de médicament pour la libération de médicament parentérale, en particulier intravasculaire, comprenant l'étape suivante :
- mélange ou revêtement de magnésium élémentaire dans une forme biocorrodable avec au moins un médicament, qui peut être présent sous forme de base ou de sel protoné correspondant à cette base, la base ayant une valeur pKb de l'ordre de 2 à 6, et éventuellement d'autres produits auxiliaires.
